# EUROPEAN PATENT APPLICATION

(11) **EP 1 767 537 A1**
(43) Date of publication of application: **28.03.2007**
(21) Application number: 05020568.1
(22) Date of filing: 21.09.2005
(51) Int. Cl.: C07D 491/04, A61K 31/519

(54) **Pyrimidine compounds for the treatment of inflammatory disorders**

(71) Applicant: Cellzome (UK) Ltd., Little Chesterford Cambridge CB10 1XL (GB)
(72) Inventor: Dyke, Hazel, Flex Meadow Harlow Essex CM 19 5TR (GB); Price, Steve, Flex Meadow Harlow Essex CM 19 5TR (GB); Cramp, Sue, Flex Meadow Harlow Essex CM 19 5TR (GB)
(74) Representative: Huhn, Michael

(57) **Abstract**

The present invention relates to novel pyrimidine compounds, for the modulation of the histamine H4 receptor and the treatment or prevention of conditions mediated by the histamine H4 receptor. The invention also relates to the preparation of such compounds.

## Description

The present invention relates to novel fused heterocyclic compounds, for the modulation of the histamine H4 receptor and the treatment or prevention of conditions mediated by the histamine H4 receptor. The invention also relates to the preparation of such compounds.

Histamine is a biogenic amine important in the regulation of different physiological processes in the body. Histamine is synthesized, by histidine decarboxylation, from L-histidine in specific cell types such as mast cells, basophils and neurons. Histamine binds to cell membrane receptors of the G-protein coupled receptors family (GPCRs). Three different histamine receptors have been first identified: H1, H2 and H3 (reviewed in S. J. Hill et al., International Union of Pharmacology. XIII. Pharmacol Rev 1997 49: 253-278). H1 receptors trigger smooth muscle contractions and play an important role in allergy. H2 receptors regulate gastric acid secretion in the stomach and H3 receptors control release of histamine and neurotransmitters by neurons. Recently a new histamine receptor (H4) has been identified and it has the highest similarity to the H3 receptor (35% overall amino acid similarity and 58% similarity in the transmembrane region) (Oda T et al., J Biol Chem 2000 Nov 24;275(47):36781-6, Zhu Y et al., Mol Pharmacol. 2001 Mar;59(3):434-41, Morse KL et al., J Pharmacol Exp Ther. 2001 Mar;296(3):1058-66, Nguyen T et al., Mol Pharmacol. 2001 Mar;59(3):427-33., Liu C et al., Mol Pharmacol. 2001 Mar;59(3):420-6, Nakamura T et al., Biochem Biophys Res Commun. 2000 Dec 20;279(2):615-20).

The H4 receptor is expressed mainly in cells of the hemopoietic lineage, especially mast cells, eosinophils and basophils (Oda T et al., J Biol Chem. 2000 Nov 24;275(47):36781-6, Zhu Y et al., Mol Pharmacol. 2001 Mar;59(3):434-41, Morse KL et al., J Pharmacol Exp Ther. 2001 Mar;296(3):1058-66, Liu C et al., Mol Pharmacol. 2001 Mar;59(3):420-6). The H4 receptor is also expressed in lower species (mouse, rat, guinea pig) and its tissue distribution is similar to human. However H4 receptor sequence in mouse, rat, and guinea pig is significantly different from the human H4 sequence (69%, 68%, 65% similarity respectively) and this translates in differences in binding affinities to histamine and H3/H4 ligands, as well as differences in signal transduction response (Liu C et al., J Pharmacol Exp Ther. 2001 Oct;299(1):121-30).

H4 receptors have been shown to mediate chemotaxis and calcium mobilization in mast cells (Hofstra CL et al., J Pharmacol Exp Ther. 2003 Jun;305(3):1212-21) and eosinophil chemotaxis with cell shape change and up regulation of adhesion molecules (Ling P et al., Br J Pharmacol. 2004 May;142(1):161-71. Erratum in: Br J Pharmacol. 2004 Jul;142(6):1052, Buckland KF et al., Br J Pharmacol. 2003 Nov; 140(6):1117-27, O'Reilly M et al., J Recept Signal Transduct Res. 2002 Feb-Nov;22(1-4):431-48). H4 is also implicated in histamine-induced interleukin-16 release from human CD8+ T cells (Gantner F et al., J Pharmacol Exp Ther. 2002 Oct;303(1):300-7), in leukotriene B4 production and mast cell dependent neutrophil recruitment (Takeshita K et al., J Pharmacol Exp Ther. 2003 Dec;307(3):1072-8). All these data indicate that the histamine H4 receptor may play a role in the inflammatory response.

An indole amide, JNJ7777120, has been recently described as a potent and selective Histamine H4 receptor antagonist, (Ki 4.5 nM. pA2 8.1. Equipotent against human, mouse and rat receptors. >1000-fold selectivity over H1, H2, or H3 receptor and no cross binding against 50 other targets) (Thurmond RL et al., J Pharmacol Exp Ther. 2004 Apr,309(1):404-13). JNJ7777120, *in vitro,* blocks histamine-induced chemotaxis and calcium influx in mouse mast cells, and *in vivo* histamine induced migration of tracheal mast cells from the connective tissue to the epithelium in mice. JNJ7777120 reduces infiltration in a mouse zymosan-induced peritonitis model indicating a role of the histamine H4 receptor in an inflammatory process *in vivo.*

Therefore it is believed that modulators of the histamine H4 receptor have utility in a variety of inflammation disorders.

Background material about inflammation can be found in the following reviews: Tracey KJ., Nature. 2002 Dec 19-26; 420(6917):853-9, Nathan C., Nature. 2002 Dec 19-26; 420(6917):846-52.

Inflammation herein refers to the response that develops as a consequence of histamine release that can be caused by immunological and non-immunological stimuli. Inflammation can be due to any one or a plurality of conditions including, but not limited to, allergy, allergic rhinitis and asthma. In terms of the development of the disorder the inflammatory conditions include, but are not limited to, acute inflammation, allergic inflammation and chronic inflammation.

There is a need in the art for new effective modulators of the histamine H4 receptor.

Thus, the object of the present invention is to provide a new class of histamine H4 receptor modulators which may be effective, preferably in the treatment of inflammatory diseases.

Accordingly, the present invention provides compounds of the formula (I) 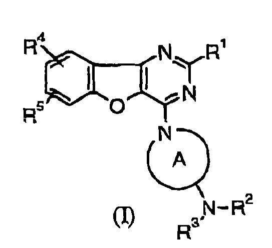 or a pharmaceutically acceptable salt, ester, prodrug or metabolite thereof, wherein

A represents heterocyclyl, having at least one nitrogen ring atom, which nitrogen is attached to the pyrimidine ring in formula (I) and wherein A is substituted with -NR²R³ and optionally substituted with one or more other substituents independently selected from the group consisting of C₁₋₄ alkyl; F, Cl, Br, C₃₋₆ cycloalkyl;
R¹ represents H, C₁₋₄ alkyl, C₃₋₆ cycloalkyl, wherein each C₁₋₄ alkyl, C₃₋₆ cycloalkyl is optionally substituted with one or more halogen;
R² and R³ are independently H, C₁₋₄ alkyl or C₃₋₆ cycloalkyl; wherein each C₁₋₄ alkyl, C₃₋₆ cycloalkyl is optionally substituted with one or more halogen;
Optionally R², R³ jointly form together with the nitrogen to which they are attached to a heterocyclyl ring;
R⁴ and R⁵ are independently H, F, Cl, Br, CN, C₁₋₄ alkyl, OH, OC₁₋₄ alkyl, C(O)OH, C(O)OC₁₋₄ alkyl, C(O)NH₂, C(O)NHC₁₋₄ alkyl, C(O)N(C₁₋₄ alkyl)₂ wherein each C₁₋₄ alkyl is optionally substituted with one or more halogen.

Within the meaning of the present invention the terms are used as follows:
"Alkyl" means a straight-chain or branched carbon chain that may contain double or triple bonds. It is generally preferred that alkyl doesn't contain double or triple bonds. Each hydrogen of an alkyl carbon may be replaced by a substituent.
"C₁₋₄ alkyl" means an alkyl chain having 1-4 carbon atoms, e.g. if present at the end of a molecule: methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl, tert-butyl, or e.g. -CH₂-, -CH₂-CH₂-, -CH=CH-, -CH(CH₃)-, -C(CH₂)₂-, -CH₂-CH₂-CH₂-, -CH₂- CH₂-CH₂-CH₂-, -CH(C₂H₅)-, -CH(CH₃)₂-, when two moieties of a molecule are linked by the alkyl group. Each hydrogen of a C₁₋₄ alkyl carbon may be replaced by a substituent.
"Heterocyclyl" or "heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine. "Heterocycle" means also azetidine.
"C₃₋₆ Cycloalkyl" means cyclopropyl, cyclopentyl, cyclohexyl and cycloheptyl.
"Halogen" means fluoro, chloro, bromo or iodo. It is generally preferred that halogen is fluoro or chloro.
"Fully saturated" pertains to compounds and/or groups which do not have any carbon-carbon, carbon-nitrogen, nitrogen-nitrogen double bonds or carbon-carbon triple bonds.

Preferred compounds are those of formula (Ia) 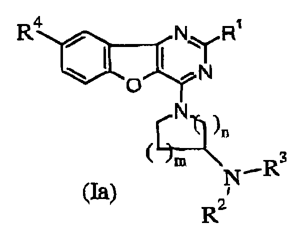 wherein n is 1 or 2; m is 0, 1 or 2 and R¹ to R⁴ as defined above.

Also preferred compounds of formula (I) and (Ia) are those compounds in which one or more of the residues contained therein have the meanings given below, with all combinations of preferred substituent definitions being a subject of the present invention.

Preferably A represents a fully saturated heterocyclic ring, preferably selected from thec group consisting of azeditine, pyrrolidine, oxazolidine, thiazolidine, pyridine, piperazine, and morpholine, more preferred azeditine or pyrrolidine.

Furthermore, it is preferred that R¹ is H or C₁₋₄ alkyl.

Preferably, R², R³ are independently H or C₁₋₄ alkyl.

Preferably R⁴, R⁵ are independently H, Cl, OH, CH₃, OCH₃, CF₃, OCF₃, C(O)OH, C(O)NH₂, or CN.

Preferred specific compounds are those selected from the group consisting of
N-[(R,S)-1-(8-Chloro-2-methylbenzo[4,5]furo[3,2-d]-pyrimidin-4-yl)-pyrrolidin-3-yl]-N-methyl amine;
N-[(R,S)-1-(8-Chlorobenzo[4,5]furo[3,2-d]-pyrimidin-4-yl)-pyrrolidin-3-yl]-N-methyl amine;
[(R,S)-1-(8-Chloro-2-methylbenzo[4,5]furo[3,2-d]pyrimidin-4-yl)pyrrolidin-3-yl] dimethyl amine;
N-[(R)-1-(8-chlorobenzo[4,5]furo[3,2-d]pyrimidin-4-yl)pyrrolidin-3-yl] N-methyl amine;
N-[(S)-1-(8-chlorobenzo[4,5]furo[3,2-d]pyrimidin-4-yl)pyrralidin-3-yl] N-methyl amine;
N-[1-(8-chlorobenzo[4,5]furo[3,2-d]pyrimidin-4-yl)azetidin-3-yl] amine;
N-[1-(8-chlorobenzo[4,5]furo[3,2-d]pyrimidin-4-yl)azetidin-3-yl] N-methyl amine; and
N-[1-(8-Chlorobenzo[4,5]furo[3,2-d]pyrimidin-4-yl)azetidin-3-yl] N,N-dimethyl amine.

Some of the compounds of the inventions and/or salts or esters thereof, will exist in different stereoisomeric forms. All of these forms are subjects of the invention.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I) or (Ia) may be obtained from stereoselective synthesis using optically pure starting materials.

Furthermore, the invention relates to any of the compounds according to the invention and/or a pharmaceutically acceptable salt or ester thereof, especially for use as a medicament.

Described below are exemplary salts of the compounds according to the invention which are included herein. The list of the different salts stated below is not meant to be complete and limiting.

Compounds according to the invention which contain one or more basic groups, i.e. groups which can be protonated can be used according to the invention in the form of their addition salts with inorganic or organic acids.

Examples for suitable salts include hydrogen chloride, hydrogen bromide, phosphoric acid, sulfuric acid, nitric acid, methanesulfonic acid, p-toluenesulfonic acid, napthalenedisulfonic acid, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfamic acid, phenylpropionic acid, gluconic acid, ascorbic acid, isonicotinic acid, citric acid, adipic acid and other acids known to a person skilled in the art.

The term "pharmaceutically acceptable" means approved by a regulatory agency such as the EMEA (Europe) and/or the FDA (US) and/or any other national regulatory agency for use in animals, preferably in humans.

The respective salts of the compounds according to the invention can be obtained by customary methods which are known to the person skilled in the art, for example by contacting these with an organic or inorganic acid or base in a solvent or dispersant, or by anion exchange or cation exchange with other salts.

Furthermore, the invention includes all salts of the compounds according to the invention which, owing to low physiological compatibility, are not directly suitable for use in pharmaceuticals but which can be used, for example, as intermediates for chemical reactions or for the preparation of pharmaceutically acceptable salts or which might be suitable for the H4 antagonist activity of a compound according of the invention in any suitable manner, such as any suitable in vitro assay.

The present invention furthermore includes all solvates of the compounds according to the invention.

The present invention furthermore includes derivatives/prodrugs (including the salts thereof) of the compounds according to the invention which contain physiologically tolerable and cleavable groups and which are metabolized in animals, preferably mammals, most preferably humans into a compound according to the invention.

The term "prodrug" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of a prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods.

The present invention furthermore includes the metabolites of the compounds according to the invention.

The term "metabolites" refers to all molecules derived from any of the compounds according to the invention in a cell or organism, preferably mammal.

Preferably the term relates to molecules which differ from any molecule which is present in any such cell or organism under physiological conditions.

The structure of the metabolites of the compounds according to the invention will be obvious to any person skilled in the art, using the various appropriate methods

Another object of the invention is a pharmaceutical composition comprising a compound according to the present invention in a mixture with an inert carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by mixing a compound of the present invention and a pharmaceutically acceptable carrier.

Any suitable route of administration may be employed for providing a mammal, especially a human, with an effective dosage of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like.

The pharmaceutical compositions of the present invention comprise a compound of formula (I) or (Ia) as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases or acids including inorganic bases or acids and organic bases or acids.

The compositions include compositions suitable for oral, rectal, topical, parenteral (including subcutaneous, intramuscular, and intravenous), ocular (ophthalmic), pulmonary (aerosol inhalation), or nasal administration, although the most suitable route in any case will depend on the nature and severity of the conditions being treated and on the nature of the active ingredient, They may be conveniently presented in unit dosage form and prepared by any of the methods well-known in the art of pharmacy.

For administration by inhalation, the compounds of the present invention are conveniently delivered in the form of an aerosol spray presentation from pressurized packs or nebulizers. The compounds may also be delivered as powders which may be formulated and the power composition may be inhaled with the aid of insufflation powder inhaler device. The preferred delivery systems for inhalation are metered dose inhalation (MDI) aerosol, which may be formulated as a suspension or solution of a compound of formula (I) or (Ia) in suitable propellants, such as fluorocarbons or hydrocarbons and dry powder inhalation (DPI) aerosol, which can be formulated as a dry powder of a compound of formula (I) or (Ia) with or without additional excipients,

Suitable topical formulations of a compound of formula (I) or (Ia) include transdermal devices, aerosols, creams, ointments, lotions, dusting powders and the like.

In practical use, the compounds of formula (I) or (Ia) can be combined as the active ingredient in intimate admixture with a pharmaceutical carrier according to conventional pharmaceutical compounding techniques. The carrier may take a wide variety of forms depending on the form of preparation desired for administration, e.g. oral or parenteral (including intravenous). In preparing the compositions for oral dosage form, any of the usual pharmaceutical media may be employed, such, as, for example, water, glycols, oils, alcohols, flavouring agents, preservatives, colouring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets, with the solid oral preparations being preferred over the liquid preparations. Because of their ease of administration, tablets and capsules represent the most advantageous oral dosage unit form in which case solid pharmaceutical carriers are obviously employed. If desired, tablets may be coated by standard aqueous or nonaqueous techniques.

In addition to the common dosage forms set out above, the compounds of formula (I) or (Ia) may also be administered by controlled release means and/or delivery devices such as those described in U.S patents 3845770, 3916899, 3536809, 3598123, 3630200 and 4008719.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or moulding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Moulded tablets may be made by moulding in a suitable machine, a mixture of the powdered compound moistened with an inert liquid diluent. Desirably, each tablet contains from about 1 mg to about 500 mg of the active ingredient and each cachet or capsule contains from about 1 to about 500 mg of the active ingredient.

The following are examples of representative pharmaceutical dosage forms for the compounds of formula (I) or (Ia):

**Injectable Suspension (I.M.):**

| | |
|---|---|
| Compound of formula (I) or (Ia) | 10 mg / mL |
| Methylcellulose | 5,0 mg / mL |
| Tween 80 | 0,5 mg / mL |
| Benzyl alcohol | 9,0 mg / mL |
| Benzalkonium chloride | 1,0 mg / mL |
| Plus water for injection to a total volume of 1mL | |

**500 mg Tablet:**

| | |
|---|---|
| Compound of formula (I), (Ia) | 25 mg / tablet |
| Microcrystalline Cellulose | 415 mg/mL |
| Povidone | 14,0 mg/mL |
| Pregelatinized Starch | 43,5 mg/mL |
| Magnesium Stearate | 2,5 mg/mL |

**600 mg Capsule:**

| | |
|---|---|
| Compound of formula (I) or (Ia) | 25 mg / tablet |
| Lactose Powder | 573,5 mg / tablet |
| Magnesium Stearate | 1,5 mg / tablet |

**Aerosol:**

| | |
|---|---|
| Compound of formula (I) or (Ia) | 24 mg / canister |
| Lecithin, NF Liq. Cone. | 1,2 mg / canister |
| Trichlorofluoromethane, NF | 4,025 g / canister |
| Dichlorodifluoromethane, NF | 12,15 g / canister |

Compounds of formula (I) or (Ia) may be used in combination with other drugs that are used in the treatment/prevention/suppression or amelioration of the diseases or conditions for which compounds of formula (I) are useful. Such other drugs may be administered, by a route and in an amount commonly used therefore, contemporaneously or sequentially with a compound of formula (I) or (Ia). When a compound of formula (I) or (Ia) is used contemporaneously with one or more other drugs, a pharmaceutical composition containing such other drugs in addition to the compound of formula (I) or (Ia) is preferred. Accordingly, the pharmaceutical compositions of the present invention include those that also contain one or more other active ingredients, in addition to a compound of formula (I) or (Ia).

Thus the present invention is also concerned with pharmaceutical compositions for preventing and treating histamine-mediated diseases comprising a therapeutically effective amount of a compound of the invention of formula (1) or (Ia) and one or more other therapeutic agents. Suitable therapeutic agents for a combination therapy with compounds of formula (I) or (Ia) include: (1) a corticosteroid, for example fluticasone or budesonide; (2) a β2-adrenoreceptor agonist, for example salmeterol or formeterol; (3) a leukotriene modulator, for example montelukast or pranlukast; (4) anticholinergic agents, for example selective muscarinic-3 (M3) receptor antagonists such as tiotropium bromide; (5) phosphodiesterase-IV (PDE-IV) inhibitors, for example roflumilast or cilomilast; (6) an antitussive agent, such as codeine or dextramorphan; (7) a non-steroidal anti-inflammatory agent (NSAID), for example ibuprofen or ketoprofen; (8) an H1 antagonist or inverse agonist, for example loratidine or cetirizine.

Another object of the present invention is a compound according to the invention for use as a medicament.

Yet another object of the present invention is the use of a compound of the present invention for the manufacture of a medicament for the treatment or prophylaxes of one or more diseases or disorders associated with the modulation of histamine H4 receptor, especially with the inflammatory response mediated by histamine H4 receptor.

Yet another object of the present invention is the use of a compound according to the present invention for the manufacture of a medicament for the treatment or prophylaxes of inflammatory diseases.

Yet another object of the present invention is the use of a compound according to the present invention for the manufacture of a medicament for the treatment or prophylaxes of one or more diseases or disorders selected from the group consisting of asthma, psoriasis, rheumatoid arthritis, Crohn's disease, inflammatory bowel disease, ulcerative colitis, allergic rhinitis and other allergic diseases, atopic dermatitis and other dermatological disorders.

Yet another object of the present invention is a method for treating, controlling, delaying or preventing in a mammalian patient in need of treatment of one or more conditions associated with the modulation of histamine H4 receptor, wherein the method comprises the administration of said patient of a pharmaceutically effective amount of a compound according to the present invention.

The weight ratio of the compound of the formula (1) to the second active ingredient may be varied and will depend upon the effective dose of each ingredient. Generally, an effective dose of each will be used.

The present invention is also concerned with processes for preparing the compounds of this invention.

The compounds of formula (I) or (Ia) of the present invention can be prepared according to the procedures of the following schemes and examples, using appropriate materials, and are further exemplified by the following specific examples. Moreover, by utilising the procedures described with the disclosure contained herein, one of ordinary skill in the art can readily prepare additional compounds of the present invention claimed herein. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds.

The compounds of the invention of formula (I) or (Ia) may be isolated in the form of their pharmaceutically acceptable salts, such as those described previously herein above. The free acid form corresponding to isolated salts can be generated by neutralisation with a suitable acid such as acetic acid and hydrochloric acid and extraction of the liberated free acid into an organic solvent followed by evaporation. The free acid form isolated in this manner can be further converted into another pharmaceutically acceptable salt by dissolution in an organic solvent followed by addition of the appropriate base and subsequent evaporation, precipitation, or crystallisation.

It may be necessary to protect reactive functional groups (e.g. hydroxy, amino, thio or carboxy) in intermediates used in the preparation of compounds of formula (I) or (Ia) to avoid their unwanted participation in a reaction leading to the formation of compounds of formula (I) or (Ia). Conventional protecting groups, for example those described by T. W. Greene and P. G. M. Wuts in "Protective groups in organic chemistry" John Wiley and Sons, 1999, may be used.

Compounds of the invention of formula (I) (the same applies for compounds of formula (Ia)) may conveniently be prepared by the reaction in an inert solvent, usually under elevated temperatures, of a cyclic amine of formula (IV) and a compound of formula (V) in which R⁶ represents a suitable leaving group; suitable leaving groups at R⁶ include chloro, bromo, alkylsulphinyl, and alkylsulphonyl. Alternatively the reaction of intermediate (V), in which R⁶ is a halo group such as chloro or bromo, with an cyclic amine intermediate of formula (IV) may be achieved in the presence of a palladium catalyst such as a mixture of palladium bis(tnfluoroacetate) and tri(*tert*-butyl)phosphine.

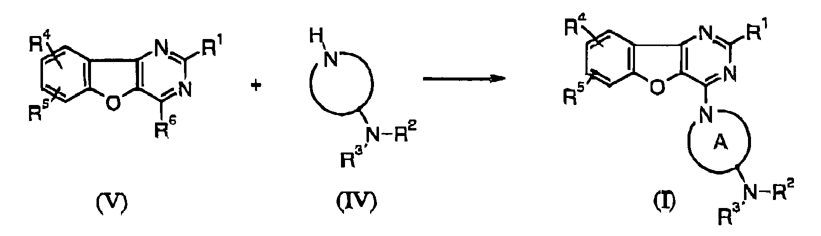

It will be understood by those practiced in the art that the transformation of intermediate (V) to compound (I) by reaction with cyclic amine (IV) when R² is H may require the presence of a suitable protecting group, for example benzyl, benzoyl or *tert-*butyloxycarbonyl, as may prove most convenient. It is to be understood that if the reaction is carried out on a protected form of cyclic amine (IV) an appropriate deprotection step will be required to obtain the desired compound (I) of the invention in which R² is H.

Intermediate compounds of formula (V) where R⁶ is chloro or bromo may be prepared, for example, by the reaction of a compound of formula (VI) with a suitable halogenating agent, for example phosphorus oxychloride or phosphorus oxybromide.

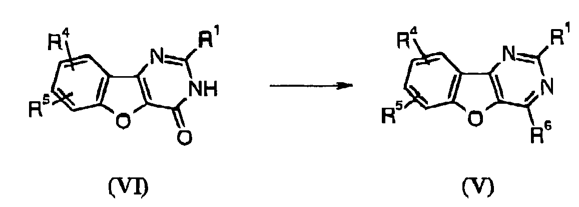

Intermediate compounds of formula (VI) may be prepared, for example, from compounds of formula (VII), where R⁷ is C(=O)NH₂, CN, or C(=O)O-alkyl, by reaction with a suitable condensing agent. Where R¹ is H suitable condensing agents include formic acid, formamide, and trialkyl orthoformates and where R¹ is alkyl or cycloalkyl suitable condensing agents include symmetrical alkyl anhydrides and alkyl amides.

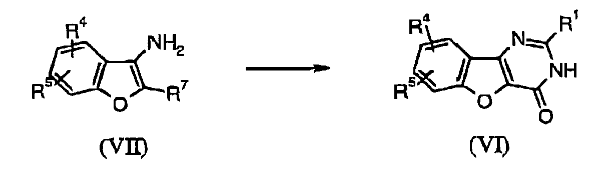

Intermediate compounds of formula (VII) may be prepared from an appropriately substituted hydroxy-benzonitrile of formula (VIII) by reaction with either bromoacetic acid ester or bromoacetonitrile in the presence of a suitable base such as potassium tertiary butoxide or sodium hydride.

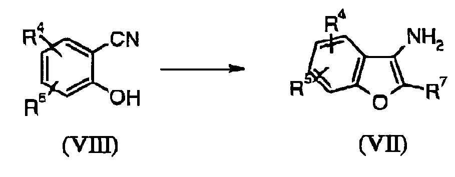

This route can serve as a basis for the preparation of compounds according to the invention by a person skilled in the art.

Another object of the present invention is a process for the preparation of a medicament comprising the steps of:
a) preparing a compound according to a process as outlined above; and
b) formulation of a medicament containing said compound.

The compounds of formula (I) and (Ia) are claimed as having activity as pharmaceuticals, in particular as modulators of histamine H4 receptor, and may be used in the treatment (therapeutic or prophylactic) of conditions/diseases in human and non-human animals, that are known to be at least partially mediated by the activation of the H4 receptor. These compounds could be beneficial for the treatment of inflammatory diseases including, but not limited to asthma, psoriasis, rheumatoid arthritis, Crohn's disease, inflammatory bowel disease, ulcerative colitis, allergic rhinitis and other allergic diseases, atopic dermatitis and other dermatological disorders.

Compounds of the invention of formula (I) and (Ia) can be tested using the following biological test methods to determine their ability to displace histamine from the H4 receptor and for their ability to antagonize the functional effects of histamine at the H4 receptor in a whole cell system.

Radioligand binding assay using histamine H4 receptor transfected CHO K1 membranes.

The receptor binding assay is performed in a final volume of 150 µL binding buffer (50 mM Tris (pH 7.4), 5 mM MgCl₂) using 18nM [2,5-3H]-histamine dihydrochloride (Amersham Biosciences UK Ltd) as the radioligand. Ligands are added in assay buffer containing a constant volume of DMSO (1% v/v). Total binding is determined using 1% v/v of DMSO in assay buffer and non-specific binding is determined using 100 µM of unlabeled histamine dihydrochloride (Sigma). The reaction is initiated with 20 µg histamine H4 receptor membranes (Euroscreen, Belgium) and the mixture incubated for 90 minutes at 25°C- The reaction is terminated by rapid filtration through GF/B filters pre-blocked with PEI (1% v/v) using a Packard Call harvester and the filter washed with 2 x 500 µL / well of cold wash buffer (50 mM Tris (pH 7.4), 5 mM MgCl₂, 0.5 M NaCl). The residual radioligand bound to the filter was determined using a Topcount liquid scintillation counter (Perkin Elmer). Compound IC₅₀ values can be determined using an 8-point dose response curve in duplicate with a semi-log compound dilution series. IC₅₀ calculations may be performed using Excel and XL fit (Microsoft) and this value can be used to determine a Kᵢ value for the test compound using the Cheng-Prusoff equation.

Functional assay using histamine H4 receptor transfected CHO K1 membranes.

The GTPγS binding assay is used as a measure of the functional activation of the histamine H4 receptor using membranes prepared from CHO K1 cells stably transfected with the cDNA for the histamine H4 receptor (Euroscreen, Belgium). The assay is performed in a 96 well Isoplate (Perkin Elmer) in a final volume of 200 µL assay buffer (20 mM HEPES (pH 7.4), 100 mM NaCl, 10 mM MgCl₂, 10 µg/ml saponin and 10 µM GDP) using 0.1nM GTPγ [35S] (Amersham Biosciences UK Ltd) to measure functional incorporation, and in the case of antagonist studies 150 nM histamine dihydrochloride (EC80 for histamine dihydrochloride) to determine maximal incorporation of GTPγ [35S]. Compounds are added in assay buffer containing a constant volume of DMSO (1 % v/v). Total incorporation is determined in the presence of 1 % v/v of DMSO in assay buffer and non-specific binding is determined using 10 µM of unlabeled GTPγS (Sigma). The incorporation is initiated with 15 µg histamine H4 receptor membranes (Euroscreen, Belgium) and the mixture incubated for 5 minutes at 30 °C. Wheat-Germ agglutinin-coated SPA beads (0.75 mg, Amersham Biosciences UK Ltd) are added and the mixture and incubated for 30 minutes at 30°C. The plate is centrifuged at 1000 x g for 10 minutes at 30 °C and radioactive incorporation counted in a MicroBeta Counter (Wallac).

### Examples

The invention will now be described in detail with reference to the following example. It will be appreciated that the invention is described by way of example only and modification of detail may be made without departing from the scope of the invention.

¹H NMR spectra were recorded at ambient temperature using either a Varian Unity Inova (400MHz) spectrometer or a Bruker Advance DRX (400MHz) spectrometer, both with a triple resonance 5mm probe. Chemical shifts are expressed in ppm relative to tetramethylsilane. The following abbreviations have been used: br = broad signal, s = singlet, d = doublet, dd = double doublet, t = triplet, q = quartet, m = multiplet.

High Pressure Liquid Chromatography - Mass Spectrometry (LCMS) experiments to determine retention times and associated mass ions were performed using the following methods:
Method A: Experiments performed on a Micromass Platform LCT spectrometer with positive ion electrospray and single wavelength UV 254um detection using a Higgins Clipeus C18 5µm 100 x 3.0mm column and a 1 mL / minute flow rate. The initial solvent system was 95% water containing 0.1% formic acid (solvent A) and 5% acetonitrile containing 0.1% formic acid (solvent B) for the first minute followed by a gradient up to 5% solvent A and 95% solvent B over the next 14 minutes. The final solvent system was held constant for a further 5 minutes.
Method B: Experiments performed on a Micromass Platform LC spectrometer with positive and negative ion electrospray and ELS/Diode array detection using a Phenomenex Luna C18(2) 30 x 4.6mm column and a 2 ml / minute flow rate. The solvent system was 95% solvent A and 5% solvent B for the first 0.50 minutes followed by a gradient up to 5% solvent A and 95% solvent B over the next 4 minutes. The final solvent system was held constant for a further 1 minute.

Microwave experiments were carried out using a Personal Chemistry Smith Synthesizer^{™}, which uses a single-mode resonator and dynamic field tuning, both of which give reproducibility and control. Temperatures from 40-250°C can be achieved, and pressures of up to 20bar can be reached.

### Example 1: Preparation of [(R,S)-1-(8-Chloro-2-methylbenzo[4,5]furo[3,2-d]pyrimidin-4-yl)pyrrolidin-3-yl]-N-methyl amine

### Intermediate A: 5-Chloro-2-cyanomethoxybenzonitrile

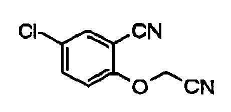

A solution of 4-chloro-2-cyanophenol (2.5g) in acetone is treated with potassium carbonate (2.3g), followed by bromoacetonitrile (1.2mL) and the resulting mixture is stirred at room temperature overnight. The mixture is filtered and the filtrate is evaporated to dryness to give 5-chloro-2-cyanomethoxybenzonitrile (3.3g) as a pale yellow solid.
¹H NMR (DMSO-d₆): δ 5.40 (s, 2H), 7.45 (d, 1H), 7.85 (dd, 1H), 8.05 (d, 1H).

### Intermediate B: 3-Amino-5-chlorobenzofuran-2-carbonitrile

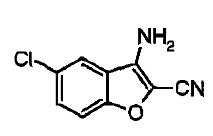

A solution of 5-cbloro-2-cyanomethoxybenzonitnle (intermediate A, 3.3g) in N,N-dimethylformamide (50mL) is treated with potassium carbonate (2.2g) and the mixture is stirred at 100°C for about 8 hours. After cooling to room temperature the reaction mixture is poured onto water and the resulting precipitate is collected by filtration, washed with water, and dried to give 3-amino-5-chlorobenzofuran-2-carbonitrile (3.0g) as a yellow solid.
¹H NMR (DMSO-d₆): δ 6,7 (br s, 2H), 7.55 (m, 2H), 8.05 (m, 1H).

### Intermediate C: 4,8-Dichloro-2-methylberkzo[4,5]furo[3,2-d]pyrimidine

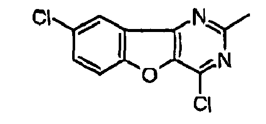

N,N-Dimethylacetamide (0.87mL) is added cautiously, under an atmosphere of nitrogen, to ice-cooled phosphorus oxychloride (2.2mL). When addition is complete the mixture is stirred with ice cooling for a further 30 minutes, then 3-amino-5-chlorobenzofuran-2-carbonitrile (intermediate B, 1.5g) is added and the resulting mixture is stirred at 50°C for 2 hours, After cooling to room temperature the mixture is evaporated to low bulk, diluted with water, and neutralised by the careful addition of solid sodium hydrogen carbonate. The mixture is extracted with chloroform (x 3), and the combined extracts are washed with water, dried over magnesium sulphate and filtered. The filtrate is evaporated to give a brown solid (1.3g). Purification of this mixture by flash chromatography eluting with a mixture of ethyl acetate and pentane (1:10) gave 4,8-dichloro-2-methylbenzo[4,5]furo[3,2-d]pyrimidine (0.16g) as a white solid.
¹H NMR (DMSO-d₆): δ 2.75 (s, 3H), 7.90 (dd, 1H), 8.00 (dd, 1H), 8.30 (dd, 1H).

### Intermediate D: tert-Butyl N-[(R,S)-1-(8-chloro-2-methylbenzo[4,5]furo[3,2-d]-pyrimidin-4-yl)-pyrrolidin-3-yl]-N-methylcarbamate

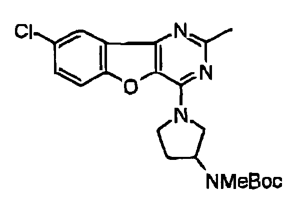

A mixture of 4,8-dichloro-2-methylbenzo[4,5]furo[3,2-d]pyrimidine (intermediate C, 0.1g), *tert*-butyl (R,S)-N-methyl-N-pyrrolidin-3-ylcarbamate (0.158g) and diethylaminomethyl polystyrene (3.2mmol/g, 0.3g) in ethanol (2mL) is irradiated in a microwave at 120°C for ten cycles of 30 seconds, cooling to 60°C between each cycle. The mixture is diluted with ethanol and filtered. The filtrate is evaporated and the residue is purified by chromatography on an Isolute® NH₂ column eluting with a mixture of ethyl acetate and cyclohexane (1:99 increasing to 1:3) to give *tert*-butyl N-[(R,S)-1-(8-chloro-2-methylbenzo[4,5]furo[3,2-d]-pyrimidin-4-yl)pyrrolidin-3-yl]-N-methylcarbamate (0.19g) as a colourless glass.
¹H NMR (CDCl₃): δ 1.5 (s, 9H), 2.15 (m, 1H), 2.25 (m, 1H), 2.65 (s, 3H), 2.85 (s, 3H), 3.7-3.95 (br, 2H), 4.15 (br, 2H), 4.95 (br, 1H), 7.45 (d, 1H), 7.5 (d, 1H), 8.15 (s, 1H).

### Product: N-[(R,S)-1-(8-Chloro-2-methylbenzo[4,5]furo[3,2-d]-pyrimidin-4-yl)-pyrrolidin-3-yl]-N-methyl amine

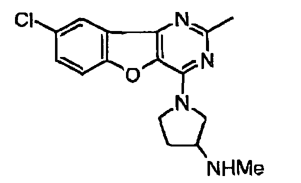

A mixture of *tert*-butyl N-[(R,S)-1-(8-chloro-2-methylbenzo[4,5]furo[3,2-d]-pyrimidin-4-yl)-pyrrolidin-3-yl]-N-methylcarbamate (intermediate D, 0.19g) and trifluoroacetic acid (4mL) in dihloromethane (4mL) is stirred at room temperature for 30 minutes. The mixture is evaporated to dryness and the residue was dissolved in acetonitrile and loaded onto an Isolute® SCX-2 column eluting with acetonitrile to remove the unwanted by-products and then with a solution of ammonia in methanol (2M). The eluant is evaporated and the residue is triturated with diethyl ether and the solid is collected by filtration and washed with diethyl ether to give a white solid. The filtrate is evaporated to dryness to give a further white solid. The solids are combined to give N-[(R,S)-1-(8-chloro-2-methylbenzo[4,5]furo[3,2-d]-pyrimidin-4-yl)-pyrrolidin-3-yl] N-methyl amine (0.049g) as a white solid.
¹H NMR (CDCl₃): δ 2.05 (m, 1H), 2.3 (m, 1H), 2.55 (s, 3H), 2.65 (s, 3H), 3.5 (m, 1H), 3.85 (br, 1H), 4.0 (br, 1H), 4.1 (br, 2H), 7.45 (d, 1H), 7.5 (d, 1H) 8.1 (s, 1H).
LCMS (method A): retention time 4.26 minutes (M+H⁺)317.

### Example 2: Preparation of [(R,S)-1-(8-Chlorobenzo[4,5]furo[3,2-d]pyrimidin-4-yl)pyrrolidin-3-yl]-N-methyl amine

### Intermediate E: Ethyl (4-chloro-2-cyanophenoxy)acetate

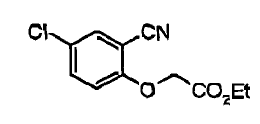

A solution of 4-chloro-2-cyanophenol (5,0g) in N,N-dimethylformamide (20mL) is added dropwise to an ice-cooled suspension of sodium hydride (60% oil dispersion, 1.4g) in N,N-dimethylformamide (165mL). The resultant mixture is stirred at 0-5°C for 40 minutes, then ethyl bromoacetate (3.9mL) is added and stirring is continued for 1.5 hours. Ethyl acetate and hydrochloric acid (1M) are added and the layers were separated. The organic layer is washed with water, aqueous sodium bicarbonate solution and brine, then dried over sodium sulphate and filtered. The filtrate is evaporated to give crude ethyl (4-chloro-2-cyanophenoxy)acetate (7.8g) as a yellow oil which is used without further purification.

### Intermediate F: Ethyl 3-amino-5-chlorobenzofuran-2-carboxylate

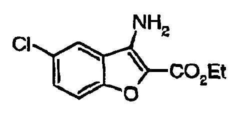

A solution of ethyl (4-chloro-2-cyanophenoxy)acetate (intermediate E, 0.9g) in dry tetrahydrofuran (5mL) is added dropwise to a suspension of potassium *tert*-butoxide (0.422g) in dry tetrahydrofuran (5mL) under an atmosphere of argon. The resultant thick mixture is stirred at room temperature for 1.5 hours. Water is added followed by acetic acid and the resultant suspension is extracted with chloroform, washed with water, aqueous sodium bicarbonate and brine, then dried over sodium sulphate and filtered. The filtrate is evaporated to give ethyl 3-amino-5-chlorobenzofuran-2-carboxylate (0.4g) as an off-white solid.
¹H NMR (CDCl₃): δ 1.45 (t, 3H), 4.45 (q, 2H), 4.95 (br s, 2H), 7.45 (s, 2H), 7.55 (s, 1H).

### Intermediate G: 8-Chloro-3H-benzo[4,5]furo[3,2-d]pyrimidin-4-one

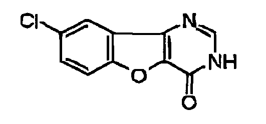

A suspension of ethyl 3-amino-5-chlorobenzofuran-2-carboxylate (intermediate F, 0.3g) in triethyl orthoformate (2mL) is irradiated in a microwave at 200°C for 10 minutes. The resultant yellow solution is evaporated to dryness and the residue was dissolved in a solution of ammonia in methanol (2M, 2mL). The mixture is carefully irradiated in a microwave at 140°C for 10 minutes and the resultant precipitate is collected by filtration and washed with diethyl ether. The filtrate is evaporated to dryness, the residue is triturated with acetonitrile and the solid was collected by filtration. The solids are combined to give 8-chloro-3H-benzo[4,5]furo[3,2-d]pynnudin-4-one (0.175 g) as a grey powder.
¹H NMR (DMSO-D₆): δ 7.6 (d, 1H), 7.9 (d, 1H), 8.1 (s, 1H). 8.25 (s, 1H).

### Intermediate H: 4,8-Dichlorobenzo[4,5]furo[3,2-d]pyrimidine

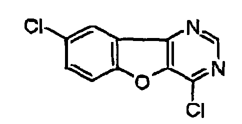

A mixture of 8-chloro-3H-benzo[4,5]ruro[3,2-d]pyrimidin-4-one (intermediate G, 0.175g) and phosphorus oxychloride (2mL) is irradiated in a microwave at 180°C for 15 minutes. The solution is evaporated to dryness and the residue was treated with aqueous ammonia solution. The resultant solid is collected by filtration and triturated with acetonitrile, sonicated with water, collected by filtration and finally triturated with acetonitrile to give 4,8-dichlorobenzo[4,5]furo[3,2-d]pyimidine (0.107g) as a beige solid.
¹H NMR (CDCl₃): δ 7.7 (d, 1H), 7.75 (d, 1H), 8.25 (s, 1H), 9.05 (s, 1H).

### Intermediate I: tert-Butyl N-[(R,S)-1-(8-chlorobenzo[4,5]furo[3,2-d]-pyrimidin-4-yl)-pyzxolidin-3-yl]-N-methylcazbamate

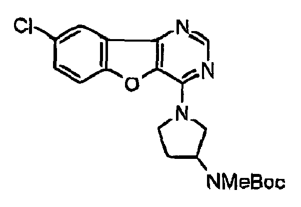

This compound is prepared from the appropriate starting materials by following a similar procedure to that used for the preparation of intermediate D and starting from 4,8-dichlorobenzo[4,5]furo[3,2-d]pyrimidine (intermediate H).
¹H NMR (CDCl₃): δ 1.5 (s, 9H), 2.15 (m, 1H), 2.25 (m, 1H), 2.85 (s, 3H), 3.65-3.95 (br, 2H), 4.2 (br, 2H), 4.95 (br, 1H), 7.5 (d, 1H), 7.55 (d, 1H), 8.1 (s, 1H), 8.6 (s, 1H).

### Product: N-[(R,S)-1-(8-Chlorobenzo[4,5]furo[3,2,-d]-pyrimidin-4-yl)-pyrrolidin-3-yl]-N-methyl amine

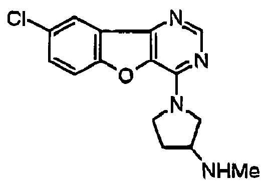

This compound is prepared starting from *tert*-butyl N-[(R,S)-1-(8-chlorobenzo[4,5]furo[3,2-d]-pyrimidin-4-yl)-pyrrolidin-3-yl]-N-methylcarbamate (intermediate I) by following a similar procedure to that used for the preparation of example 1.
¹H NMR (CDCl₃): δ 2.0 (m, 1H),2.3 (m, 1H), 2.55 (s, 3H), 3.5 (m, 1H), 3.85 (br, 1H), 4.0 (br, 1H), 4.1 (br, 2H), 7.5 (d, 1H), 7.55 (d, 1H), 8.1 (s, 1H), 8.55 (s, 1H).
LCMS (method A): retention time 5.15 minutes (M+H⁺) 303.

### Example 3: [(R,S)-1-(8-Chloro-2-methylbenzo[4,5]furo[3,2-d]pyrimidin-4-yl)pyrrolidin-3-yl] dimethyl amine

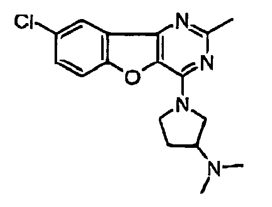

A mixture of 4,8-dichloro-2-methylbenzo[4,5]furo[3,2-d]pyrimidine (intermediate C, 0.1g), (R,S)-3-dimethylaminopyrrolidine (0.09g) and diethylaminomethyl polystyrene (3.2mmol/g, 0.25g) in ethanol (2mL) is irradiated in a microwave at 120°C for ten cycles of 30 seconds, cooling to 60°C between each cycle. The mixture is diluted with ethanol and filtered. The filtrate is evaporated and the residue is purified by chromatography on silica eluting with a mixture of ethyl acetate and cyclohexane (1:99 increasing to 99:1) to give [(R,S)-1-(8-chloro-2-methylbenzo[4,5]furo[3,2-d]pyrimidin-4-yl)pyrrolidin-3-yl] dimethyl amine (0.09g) as a white solid.
¹H NMR (CDCl₃): δ 1.95 (m, 1H) 2.3 (m, 1H), 2.4 (s, 6H), 2.7 (s, 3H), 2.9 (m, 1H), 3.6 (br, 1H), 3.85 (br, 1H), 4.25 (br, 2H), 7.45 (d, 1H), 7.5 (d, 1H), 8.15 (s, 1H).
LCMS (method A): retention time 4.28 minutes (M+H⁺) 331.

### Example 4: Preparation of N-[(R)-1-(8-chloro-benzo[4,5]furo[3,2-d]pyrimidin-4-yl)pyrrolidin-3-yl] N-methyl amine

### Intermediate J: tert-Butyl N-[(R)-1-(8-chlorobenzo[4,5]furo[3,2-d]pyrimidin-4-yl)pyrrolidin-3-yl] carbamate

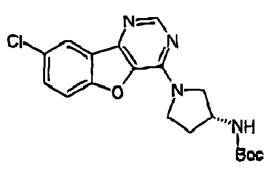

This compound is prepared starting from 4,8-dichlorobenzo[4,5]furo[3,2-d]pyrimidine (intermediate H) and *tert*-butyl (R)-N-pyrrolidin-3-ylcarbamate by following a similar procedure to that used for the preparation of intermediate D.
¹H NMR: (CDCl₃) δ 1.45 (s, 9H), 2.1 (br, 1H), 2.35(m, 1H), 3.9 (br, 1H), 4.05 (br, 1H), 4.3 (br, 1H), 4.45 (br, 1H), 4.8 (br, 1H), 7.5 (d, 1H), 7,55 (d, 1H), 8.2 (br s, 1H), 8.55 (s, 1H).

### Intermediate K: tert-Butyl N-[(R)-1-(8-chlorobenzo[4,5]furo[3,2-d]pyrimidin-4-yl)pyrrolidin-3-yl] N-methyl carbamamte

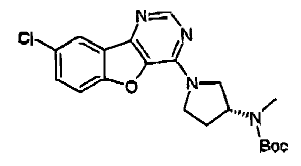

A mixture of *tert*-butyl N-[(R)-1-(8-chlorobenzo[4,5]furo[3,2-d]pyrimidin-4-yl)pyrrolidin-3-yl] carbamate (intermediate J, 0.109g) and sodium hydride (60% oil dispersion, 0.05g) in dry tertrahydrofuran (3mL) is sonicated under an atmosphere of argon for 10 minutes. Iodomethane (1mL) is added and the mixture was sonicated for a further 1 hour. The mixture is treated with a saturated solution of ammonium chloride and the resultant mixture si extracted with ethyl acetate, dried over magnesium sulphate and filtered. The filtrate is evaporated to give *tert*-butyl N-[(R)-1-(8-chlorobenzo[4,5]furo[3,2-d]pyrimidin-4-yl)pyrrolidin-3-yl] N-methyl carbamate (0.092g) as an off-white solid,
¹H NMR: (CDCl₃) δ 1.5 (s, 9H), 2.2 (m, 1H), 2.25 (m, 1H), 2.9 (s, 3H), 3.8 (br, 2H), 4.2 (br, 2H), 5.0 (br, 1H), 7.5 (d, 1H), 7.55 (d, 1H), 8.15 (s, 1H), 8.6 (s, 1H).

### Product: N-[(R)-1-(8-chlorobenzo[4,5]furo[3,2-d]pyrimidin-4-yl)pyrrolidin-3-yl] N-methyl amine

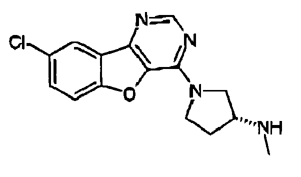

This compound is prepared starting from *tert*-butyl N-[(R)-1-(8-chlorobenzo[4,5]furo[3,2-d]pycimidin-4-yl)pyxrolidin-3-yl] N-methyl carbamamte (intermediate K) following a similar procedure to that used for the preparation of example 1.
¹H NMR: (CD₃OD) 2.0 (br, 1H), 2.3 (m, 1H), 2.5 (s, 3H), 3.5 (m, 1H), 3.6-4.4 (br, 4H), 7.6 (d, 1H), 7.65 (d, 1H), 8.05 (s, 1H), 8.4 (s, 1H).
LCMS (method A): retention time 5.2 minutes (M+H⁺) 303.

### Example 5: Preparation of N-[(S)-1-(8-chlorobenzo[4,5]furo[3,2-d]pyrimidin-4-yl)pyrrolidin-3-yl] N-methyl amine

### Intermediate L: tert-Butyl N-[(S)-1-(8-chlorobenzo[4,5]furo[3,2-d]pyrimidin-4-yl)pyrrolidin-3-yl] carbamate

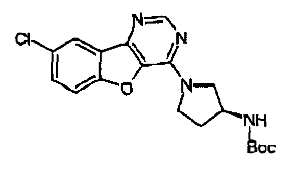

This compound is prepared starting from 4,8-dichlorobenzo[4,5]furo[3,2-d]pyrimidine (intermediate H) and *tert*-butyl (S)-N-pyrrolidin-3-ylcarbamate by following a similar procedure to that used for the preparation of intermediate D.
¹H NMR: (CDCl₃) δ 1.45 (s, 9H), 2.1 (br, 1H), 2.35(m, 1H), 3.9 (br, 1H), 4.05 (br, 1H), 4.3 (br, 1H), 4.45 (br, 1H), 4.8 (br, 1H), 7.5 (d, 1H), 7.55 (d, 1H), 8.2 (br s, 1H), 8.55 (s, 1H).

### Intermediate M: tert-Butyl N-[(S)-1-(8-cblorobenzo[4,5]furo[3,2-d]pyrimidin-4-yl)pyrrolidin-3-yl] N-methyl carbamamte

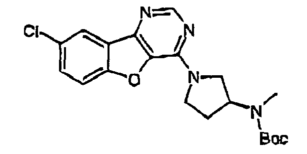

This compound is prepared starting from *tert*-butyl N-[(S)-1-(8-chlorobenzo[4,5]furo[3,2-d]pyrnoidin-4-yl)pynolidin-3-yl] carbamate (intermediate L) following a similar procedure to that used for the preparation of intermediate K.
¹H NMR: (CDCl₃) δ 1.5 (s, 9H), 2.2 (m, 1H), 2.25 (m, 1H), 2.9 (s, 3H), 3.8 (br, 2H), 4.2 (br, 2H), 5.0 (br, 1H), 7.5 (d, 1H), 7.55 (d, 1H), 8.15 (s, 1H), 8.6 (s, 1H).

### Product: N-[(S)-1-(8-Chlorobenzo[4,5]furo[3,2-d]pyrimidin-4-yl)pyrrolidin-3-yl] N-methyl amine

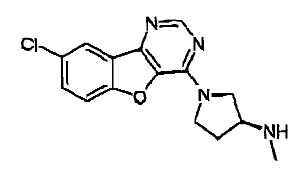

This compound is prepared starting from *tert*-butyl N-[(S)-1-(8-chlorobenzo[4,5]furo[3,2-d]pyrimidin-4-yl)pyrrolidin-3-yl] N-methyl carbamate (intermediate M) following a similar procedure to that used for the preparation of example 1.
¹H NMR: (CD₃OD) 2.0 (br, 1H), 2.3 (m, 1H), 2.5 (s, 3H), 3.5 (m, 1H), 3.6-4.4 (br, 4H), 7.6 (d, 1H), 7.65 (d, 1H), 8.05 (s, 1H), 8.4 (s, 1H).
LCMS (method A): retention time 5.18 minutes (M+H⁺) 303.

### Example 6: Preparation of N-[1-(8-chlorobenzo[4,5]furo[3,2-d]pyrimidm-4-yl)azetidin-3-yl] amine

### Intermediate N: tert-Butyl N-[1-(8-chlorobenzo[4,5]furo[3,2-d]pyrimidin-4-yl)azetidin-3-yl] carbamate

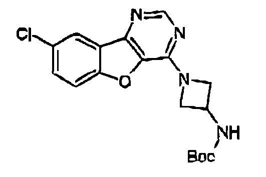

This compound is prepared starting from 4,8-dichlorobenzo[4,5]furo[3,2-d]pyrimidine (intermediate H) and *tert*-butyl N-azetidin-3-ylcarbamate by following a similar procedure to that used for the preparation of intermediate D.
¹H NMR: (CDCl₃) δ 1.5 (s, 9H), 4.3 (m, 2H), 4.8 (m. 3H), 5.2 (br, 1H), 7.5 (d, 1H), 7.55 (d, 1H), 8.15 (s, 1H), 8.55 (s, 1H).

### Product: N-[1-(8-chlorobenzo[4,5]furo[3,2-d]pyrimidin-4-yl)azetidin-3-yl] amine

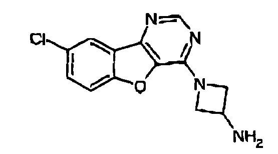

This compound is prepared starting from *tert*-butyl N-[1-(8-chlorobenzo[4,5]furo[3,2-d]pyrimidin-4-yl)azetidin-3-yl] carbamate (intermediate N) by following a procedure similar to that used for the preparation of example 1.
¹H NMR: (CD₃OD) δ 4.15 (m, 1H), 4.25 (br, 2H), 4.7 (br, 2H), 7.65 (s, 2H), 8.05 (s, 1H), 8.45 (s, 1H).
LCMS (method A) retention time 5.04 minutes (M+H⁺) 275.

### Example 7: Preparation of N-[1-(8-chlorobenzo[4,5]furo[3,2-d]pyrimidin-4-yl)azetidin-3-yl] N-methyl amine

### Intermediate O: tert-Butyl N-[1-(8-chlorobenzo[4,5]furo[3,2-d]pyrimidin-4-yl)azetidin-3-yl] N-methyl carbamate

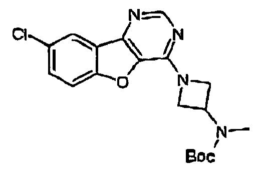

Sodium hydride (60% oil dispersion , 0.011g) is added to a solution of *tert*-butyl N-[1-(8-chlorobenzo[4,5]furo[3,2-d]pynmidin-4-yl)azetidin-3-yl] carbamate (intermediate N, 0.05g) in dry tetrahydrofuran (2mL) and the mixture was stirred for 45 minutes. Iodomethane (0.041mL) is added and the mixture is heated in a microwave at 90°C for 10 minutes. A further quantity of iodomethane is added (4 drops) and the mixture is further heated in the microwave at 90°C for 5 minutes. The mixture is diluted with water and extracted with ethyl acetate, dried over magnesium sulphate and filtered. The filtrate is evaporated and the residue is purified by chromatography on silica eluting with a mixture of ethyl acetate and cyclohexane (1:2) to give *tert*-butyl N-[1-(8-chlorobenzo[4,5]furo[3,2-d]pyrimidin-4-yl)azetidin-3-yl] N-methyl carbamate (0.025g) as a white solid.
LCMS (method B): retention time 3.47 minutes (M+H⁺) 389.

### Product: N-[1-(8-chlorobenzo[4,5]furo[3,2-d]pyrimidin-4-yl)azetidin-3-yl] N-methyl amine

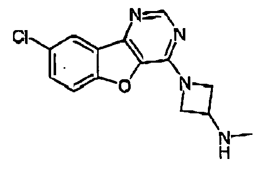

This compound is prepared starting from *tert*-butyl N-[1-(8-chlorobenzo[4,5]furo[3,2-d]pyrimidin-4-yl)azetidin-3-yl] N-methyl carbamate (intermediate O) by following a similar procedure to that used for the preparation of example 1.
¹H NMR: (CD₃OD) δ 2.4 (s, 3H), 3.9 (m, 1H), 4.25 (br, 2H), 4.7 (br, 2H), 7.65 (d, 1H), 7.7 (d, 1H), 8.1 (s, 1H), 8.45 (s, 1H).
LCMS (method A): retention time 5.15 minutes (M+H⁺) 289.

### Example 8: N-[1-(8-Chlorobenzo[4,5]furo[3,2-d]pyrimidin-4-yl)azetidin-3-yl] N,N-dimethyl amine

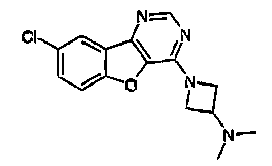

A mixture of *tert*-butyl N-[1-(8-chlorobenzo[4,5]furo[3,2-d]pyrimidin-4-yl)azetidin-3-yl] N-methyl carbamate (intermediate O, 0.022g), formaldehyde (37% aqueous solution, 0.045mL) and formic acid (0.5mL) is heated in the microwave at 150°C for 5 minutes. The resultant mixture is dissolved in methanol and loaded onto an Isolute ® SCX-2 column eluting with methanol to remove the unwanted by-products and then with a solution of ammonia in methanol (2M) to give N-[1-(8-chlorobenzo[4,5]furo[3,2-d]pyrimidin-4-yl)azetidin-3-yl] N,N-dimethyl amine (0.012g) as an off-white solid.
¹H NMR: (CDCl₃) δ 2.3 (s, 6H), 3.4 (m, 1H), 4.35 (m, 2H), 4.5 (m, 2H), 7.5 (d, 1H), 7.55 (d, 1H), 8.1 (s. 1H), 8.6 (s, 1H).
LCMS (method A): retention time 5.32 minutes (M+H⁺) 303.

### Example 9: Radioligand binding assay

The title compounds of examples 1 to 8 are tested in the radioligand binding assay using histamine H4 receptor transfected CHO K1 membranes as described above.
Compounds of the invention typically demonstrate Kᵢ values in this assay of ≤ 10 µM.

## Claims

1. A compound having the general formula (I) 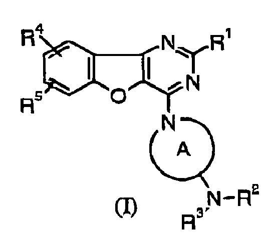 or a pharmaceutically acceptable salt, ester, prodrug or metabolite thereof, wherein
A represents heterocyclyl, having at least one nitrogen ring atom, which nitrogen is attached to the pyrimidine ring in formula (I) and wherein A is substituted with -NR²R³ and optionally substituted with one or more other substituents independently selected from the group consisting of C₁₋₄ alkyl; F, Cl, Br, C₃₋₆ cycloalkyl;
R¹ represents H, C₁₋₄ alkyl, C₃₋₆ cycloalkyl, wherein each C₁₋₄ alkyl, C₃₋₆ cycloalkyl is optionally substituted with one or more halogen;
R² and R³ are independently H, C₁₋₄ alkyl or C₃₋₆ cycloalkyl; wherein each C₁₋₄ alkyl, C₃₋₆ cycloalkyl is optionally substituted with one or more halogen;
Optionally R², R³ jointly form together with the nitrogen to which they are attached to a heterocyclyl ring;
R⁴ and R⁵ are independently H, F, Cl, Br, CN, C₁₋₄ alkyl, OH, OC₁₋₄ alkyl, C(O)OH, C(O)OC₁₋₄ alkyl, C(O)NH₂, C(O)NHC₁₋₄ alkyl, C(O)N(C₁₋₄ alkyl)₂ wherein each C₁₋₄ alkyl is optionally substituted with one or more halogen.

2. A compound according to claim 1, wherein A represents a fully saturated heterocyclic ring, preferably selected from the group consisting of azeditine, pyrrolidine, oxazolidine, thiazolidine, pyridine, piperazine, and morpholine, more preferred azeditine or pyrrolidine.

3. A compound according to claim 1 or 2, wherein
R¹ is H or C₁₋₄ alkyl;
R², R³ are independently H or C₁₋₄ alkyl;
R⁴, R⁵ are independently H, Cl, OH, CH₃, OCH₃, CF₃, OCF₃, C(O)OH, C(O)NH₂, or CN.

4. A compound according any of claims 1 to 3 having the general formula (Ia) 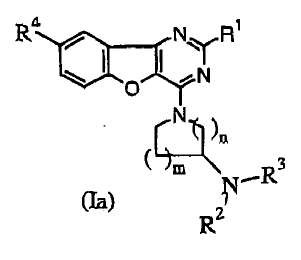 wherein n is 1 or 2; m is 0, 1 or 2 and R¹ to R⁴ as defined in claim 1.

5. A compound according to any of claims 1 to 4 selected from the group consisting of:
N-[(R,S)-1-(8-Chloro-2-methylbenzo[4,5]furo[3,2-d]-pyrimidin-4-yl)-pyrrolidin-3-yl]-N-methyl amine;
N-[(R,S)-1-(8-Chlorobenzo[4,5]furo[3,2-d]-pyrimidin-4-yl)-pyrrolidin-3-yl]-N-methyl amine;
[(R,S)-1-(8-Chloro-2-methylbenzo[4,5]furo[3,2-d]pyrimidin-4-yl)pyrrohdin-3-yl] dimethyl amine;
N-[(R)-1-(8-chlorobenzo[4,5]furo[3,2-d]pyrimidin-4-yl)pyrrolidin-3-yl] N-methyl amine;
N-[(S)-1-(8-chlorobenzo[4,5]furo[3,2-d]pyrimidin-4-yl)pyrrolidin-3-yl) N-methyl amine;
N-[1-(8-chlorobenzo[4,5]furo[3,2-d]pyrimidin-4-yl)azetidin-3-yl] amine;
N-[1-(8-chlorobenzo[4,5]furo[3,2-d]pyrimidin-4-yl)azetidin-3-yl] N-methyl amine; and
N-[1-(8-Chlorobenzo[4,5]furo[3,2-d]pyrimidin-4-yl)azetidin-3-yl] N,N-dimethyl amine.

6. A pharmaceutical composition comprising a compound of any of claims 1 to 5 in a mixture with an inert carrier.

7. A compound according to any of claims 1 to 5 for use as a medicament.

8. Use of a compound according to any of claims 1 to 5 for the manufacture of a medicament for the treatment or prophylaxes of one or more diseases or disorders associated with the modulation of histamine H4 receptor, especially with the inflammatory response mediated by histamine H4 receptor.

9. Use of a compound according to any of claims 1 to 5 for the manufacture of a medicament for the treatment or prophylaxes of inflammatory diseases.

10. Use of a compound according to any of claims 1 to 5 for the manufacture of a medicament for the treatment or prophylaxes of one or more diseases or disorders selected from the group consisting of asthma, psoriasis, rheumatoid arthritis, Crohn's disease, inflammatory bowel disease, ulcerative colitis, allergic rhinitis and other allergic diseases, atopic dermatitis and other dermatological disorders.

11. A method for treating, controlling, delaying or preventing in a mammalian patient in need of treatment of one or more conditions associated with the modulation of histamine H4 receptor, wherein the method comprises the administration of said patient of a pharmaceutically effective amount of a compound according to any of claims 1 to 5.

12. A process for the preparation of a compound according to any of the claims 1 to 5, comprising the steps of:
• Reacting an appropriately substituted hydroxyl-benzonitrile of formula (VIII) with either bromoacetic acid ester or bromoacetonitrile under conditions to form a benzofuran of formula (VII). 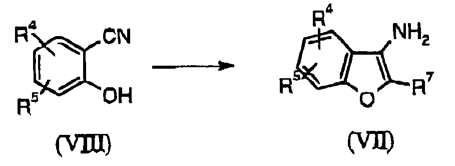
wherein R⁷ = C(=O)NH₂, CN, or C(=O)O-alkyl;
• Reacting a compound of formula (VII), with a suitable condensing agent to form a compound of formula (VI) 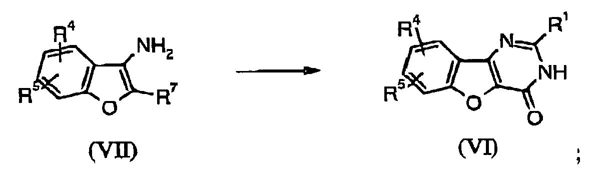
• Treating a compound of formula (VI) with a suitable halogenating agent, for example phosphorus oxychloride or phosphorus oxybromide to form activated halides of the formula (V). 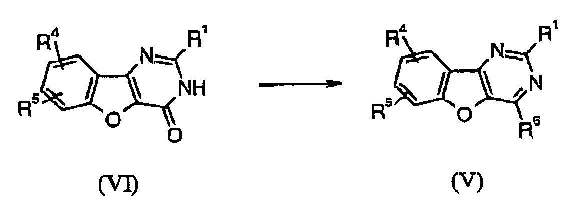
wherein R⁶ is Cl or Br;
• Reacting a cyclic amine of formula (IV) with a compound of formula (V) under conditions to form a compound of general formula (I)

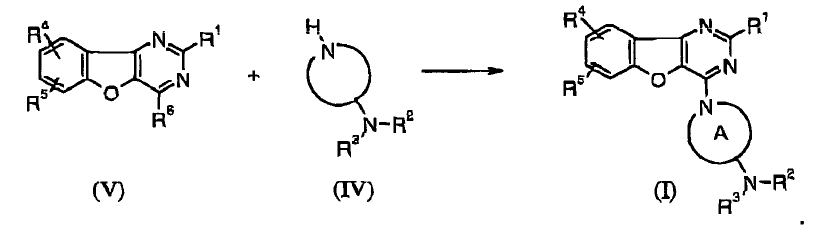

13. A process for the preparation of a medicament comprising the steps of:
a) preparing a compound according to claim 12; and
b) formulating a medicament containing said compound.
